# EUROPÄISCHE PATENTSCHRIFT

(11) **EP 1 910 841 B1**
(45) Veröffentlichungstag und Bekanntmachung des Hinweises auf die Patenterteilung: **29.06.2011**
(21) Anmeldenummer: 06762778.6
(22) Anmeldetag: 24.07.2006
(51) Int. Cl.: G01N 33/68

(54) **IN VITRO VERFAHREN ZUR DIAGNOSE VON NEURODEGENERATIVEN ERKRANKUNGEN**
IN VITRO PROCESS FOR DIAGNOSIS OF NEURODEGENERATIVE DISEASES
PROCEDE IN VITRO POUR DIAGNOSTIQUER DES MALADIES NEURODEGENERATIVES

(30) Priorität: 01.08.2005 DE 102005036094
(43) Veröffentlichungstag der Anmeldung: 16.04.2008
(73) Patentinhaber: B.R.A.H.M.S GmbH, 16761 Hennigsdorf (DE)
(72) Erfinder: BERGMANN, Andreas, 12351 Berlin (DE); ERNST, Andrea, 16761 Hennigsdorf (DE); HAMPEL, Harald, 80331 München (DE)
(74) Vertreter: Andrae, Steffen
(86) Internationale Anmeldenummer: PCT/EP2006/007272
(87) Internationale Veröffentlichungsnummer: WO 2007/014667

(56) Entgegenhaltungen:
- EP-A1- 1 867 734
- WO-A-2004/090546
- DE-A1-102005 036 094
- FRANK RICHARD A ET AL: "Biological markers for therapeutic trials in Alzheimer's disease Proceedings of the biological markers working group; NIA initiative on neuroimaging in Alzheimer's disease." NEUROBIOLOGY OF AGING, Bd. 24, Nr. 4, Juli 2003 (2003-07), Seiten 521-536, XP002406248 ISSN: 0197-4580 in der Anmeldung erwähnt
- TEUNISSEN C E ET AL: "Biochemical markers related to Alzheimer's dementia in serum and cerebrospinal fluid" NEUROBIOLOGY OF AGING, Bd. 23, Nr. 4, Juli 2002 (2002-07), Seiten 485-508, XP002406249 ISSN: 0197-4580 in der Anmeldung erwähnt

## Beschreibung

Die vorliegende Erfindung betrifft ein neues *in vitro* Verfahren für die Diagnose von neurodegenerativen Erkrankungen, insbesondere von Demenzerkrankungen wie der Alzheimer Krankheit.

Im Rahmen der vorliegenden Erfindung wird dabei der Begriff "Diagnose" als Oberbegriff für medizinische Bestimmungen gebraucht, denen je nach dem klinischen Zustand des Patienten, bei dem die Bestimmung durchgeführt wird, unterschiedliche Fragestellungen zugrunde liegen können und die insbesondere der Erkennung und Früherkennung, der Bestimmung des Schweregrads und der Verlaufsbeurteilung, auch der therapiebegleitenden Verlaufsbeurteilung, und der Prognose des zukünftigen Verlaufs einer Erkrankung dienen. Dabei ist im vorliegenden Zusammenhang von besonderer Bedeutung, dass eine Diagnose auch eine Negativdiagnose sein kann, bei der man aufgrund der Nichtfeststellbarkeit eines bestimmten krankheitstypischen Merkmals, z.B. der Nichtnachweisbarkeit eines mit der betreffenden Krankheit assoziierten Biomarkers in einer Blutprobe eines Patienten, das Vorliegen einer bestimmten Erkrankung zuverlässig ausschließt.

Für die Negativdiagnose sind auch Biomarker von großem Wert, die bei mehreren verschiedenen Krankheiten erhöht gefunden werden können und daher allein, für sich genommen noch keine positive Diagnose einer spezielle Krankheit ermöglichen - obwohl sie in der Regel bei Hinzuziehung weiterer klinischer oder biochemischer Parameter auch für die Positivdiagnose entscheidend sein können.

Die Erkrankungen, um deren Diagnose es bei der vorliegenden Erfindung geht, sind sich eher langsam entwickelnde, chronische neurodegenerative Erkrankungen von nichtinfektiöser Ätiologie, insbesondere präsenile Demenz-Erkrankungen.

Als Demenz-Erkrankungen (Dementia) werden generell Krankheiten bezeichnet, für die ein gemeinsames Merkmal der Verlust erworbener intellektueller Fähigkeiten, v.a. des Gedächtnisses, und des normalen Persönlichkeitsniveaus als Folge von Hirnschädigungen ist. Demenzerkrankungen sind in der Regel sich relativ langsam entwickelnde Krankheiten von chronischem Charakter. Treten Demenzerscheinungen vor dem hohen Alter im mittleren Lebensalter auf, werden sie als präsenile Demenz-Erkrankungen bezeichnet, und bei diesen unterscheidet man auf der Basis der für sie typischen Symptome und hirnpathologischen Veränderungen insbesondere die folgenden Erkrankungen bzw. Gruppen von Erkrankungen:

Die **Alzheimer Demenz (AD)** (Alzheimer Krankheit; Morbus Alzheimer) ist die häufigste neurodegenerative Demenz-Erkrankung und macht 2/3 aller Demenzfälle aus. AD zeichnet sich durch drei wichtige, allerdings erst post *mortem* mit Sicherheit feststellbare pathologische Merkmale aus: die Bildung von Amyloid-Plaques und neurofibrillären Bündeln sowie den Verlust an Nervenzellen (Übersicht s. 19; Literaturangaben in der Beschreibung in Form von Zahlen beziehen sich auf die der Beschreibung folgende Literaturliste). Amyloid-Plaques bestehen aus extraneuronalen Aggregaten des Amyloid-β-Proteins, während die Neurofibrillenbündel hauptsächlich Tau-Protein und Neurofilamente enthalten. Es wird vermutet, dass die Plaque- und Neurofibrillenbildung die Ursache für das Absterben von Nervenzellen ist.

Die wichtigsten Symptome von AD sind zunehmende Merkfähigkeits- und Denkstörungen bei relativ lang anhaltender Gemütsansprechbarkeit, wobei diese Symptome von weiteren weniger spezifischen Störungen begleitet werden, die die Abgrenzung der AD von anderen Demenzformen erschweren.

Die **Demenz mit Lewy-Körperchen** (dementia with lewy-bodies: DLB) ist nach der Alzheimer Demenz die zweithäufigste Ursache für eine demenzielle Erkrankung. Neuropathologisch ist die DLB durch das Auftreten von sogenannten Lewy-Körperchen im Hirnstamm und im Cortex charakterisiert. Diese LewyKörperchen bestehen überwiegend aus Aggregaten des präsynaptischen Proteins (α-Synuclein) und Ubiquitin. Die LewyBody-Pathologie kann in unterschiedlichem Ausmaß mit Alzheimer und Parkinson-typischen neuropathologischen Veränderungen assoziiert sein. So kommt es auch bei der DLB zur Bildung von beta-Amyloid und senilen Plaques, jedoch nicht zu Neurofibrillenbündeln (Übersicht s. 14). Lewy-Körperchen sind auch im Gehirn von Patienten mit Morbus Parkinson vorhanden, wenn auch in einer unterschiedlichen Verteilung.

Kernsymptome von DLB sind eine progrediente kognitive Störung, Verwirrtheitsepisoden mit fluktuierender Aufmerksamkeits- und Bewusstseinslage, Parkinsonismus, häufige Stürze und Synkopen (anfallsartige, kurz dauernde Bewusstlosigkeit). Die Sensitivität und Spezifität der diagnostischen Kriterien zeigen durchgehend eine hohe Spezifität, aber zum Teil eine sehr niedrige Sensitivität. Das bedeutet, dass die DLB im klinischen Alltag häufig nicht diagnostiziert wird.

Die **Frontotemporale Demenz (FTD)** wird auch als Pick'sche Krankheit bezeichnet und macht ca. 20% der präsenilen Demenzerkrankungen aus. FTD ist teilweise genetisch bedingt und zählt zu den sogenannten Tauopathien, die sich durch eine Über- oder Unterexpression eines Tauprotein-Subtyps bzw. durch die Expression eines mutierten Tauproteins auszeichnen. Neuropathologisch kommt es zu einer lokalen Atrophie des frontalen und/oder temporalen Cortex sowie der Substantia Nigra und der Basalganglien. Dies hat unterschiedlich ausgeprägte Sprachstörungen, eine Wesensänderung sowie Verhaltensauffälligkeiten zur Folge. Insgesamt ist die FTD mit einer Sensitivität von 93% bei einer Spezifität von nur 23% unterdiagnostiziert, wobei die AD die häufigste Fehldiagnose darstellt.

Unter dem Begriff **vaskuläre Demenz (vascular dementia; VAD)** werden Erkrankungen zusammengefasst, bei denen eine Demenz aufgrund von Durchblutungsstörungen im Gehirn ausgelöst wird. Es gibt unterschiedliche Typen der VAD, von denen die Multi-Infarkt-Demenz (MID) und die subcorticale VAD (auch als Binswanger'sche Erkrankung bezeichnet) die häufigsten Formen darstellen.

Bei der Binswanger'schen Erkrankung handelt es sich um eine langsam progrediente demenzielle Entwicklung, die pathologisch durch cerebrovasculäre Läsionen in der weißen Hirnsubstanz charakterisiert ist. Klinisch resultiert dies in Verhaltensauffälligkeiten wie Agitation, Reizbarkeit, Depression und Euphorie sowie einer leichten Gedächtnisstörung.

Die Multi-Infarkt-Demenz entsteht allmählich als Folge von mehreren kleinen Schlaganfällen, auch als transiente ischämische Attacken (TIA) bezeichnet, die zum Untergang von Hirngewebe im Cortex und/oder subcortikalen Arealen führten. Die Schlaganfälle können auch gänzlich unbemerkt geblieben sein, die Demenz ist in diesem Falle die erste spürbare Folge. Bei Vorliegen einer MID kommt es zur stufenweisen Abnahme kognitiver Fähigkeiten, verbunden mit schweren Depressionen, Stimmungsschwankungen und Epilepsie.

Eine Diagnose auf Demenz-Erkrankungen erfolgt heutzutage überwiegend auf der Basis neuropsychologischer Untersuchungen und der Beobachtung der Krankheitsentwicklung und ihres Verlaufs, unter Heranziehung von Ausschlusskriterien für bestimmte Demenzformen. Diese Untersuchungen liefern in sehr vielen Fällen mehrdeutige Ergebnisse, die die o.g. Zahlen für die unterdiagnostizierten Demenzformen oder unrichtig diagnostizierten Fälle erklären. Die krankheitstypischen Gehirnveränderungen können an lebenden Patienten naturgemäß nicht direkt festgestellt werden, und apparatemedizinische Untersuchungen der Gehirnfunktionen mittels z.B. Röntgen- oder Kernspin-Tomographie sind aufwändig und teuer.

Für die Alzheimer-Diagnostik veröffentlichten das Ronald und Nancy Reagan Institut der Alzheimer Association und die NIA-Working Group Richtlinien für die Kriterien, die an einen idealen Biomarker zur Detektion der AD gestellt werden (7). Folgende Kriterien sollen im Idealfall durch den Biomarker erfüllt werden:
1. Er sollte hirnspezifisch sein und ein fundamentales Merkmal der Neuropathologie dieser Erkrankungen detektieren.
2. Die diagnostische Sensitivität und die Spezifität von mindestens 80% sollte gegeben sein.
3. Die krankheitsspezifische Veränderung des Biomarkers sollte sich in einem möglichst frühen Stadium der Erkrankung manifestieren, um mit geeigneten Therapiemaßnahmen beginnen zu können (9).

Bis jetzt gibt es jedoch keinen Biomarker, der im klinischen Alltag im Blut oder der cerebrospinalen Flüssigkeit mit ausreichender Sicherheit zur Früh- und Differentialdiagnose von AD herangezogen werden könnte und alle o.g. Kriterien erfüllt. Aktuell werden verschiedene potentielle Markerkandidaten untersucht, darunter Inflammationsmarker wie IL-6 und TNFα, Marker für oxidativen Stress wie 3-Nitrotyrosin, sowie Marker, die mit charakteristischen pathologischen Veränderungen der AD assoziiert sind, wie Amyloid β, das einen Hauptbestandteil der Amyloid-Plaques darstellt, und das Tau-Protein, das einen wesentlichen Bestandteil der Neurofibrillenbündel darstellt (vgl. die Übersicht in 7; 26).

Es besteht ein aktueller Bedarf nach ergänzenden, valide Laborbefunde liefernden Untersuchungsmethoden, die auf einer Bestimmung von als Biomarker für Demenzerkrankungen, insbesondere für die Alzheimer Demenz (AD), geeigneten Substanzen in Blut- bzw. Plasmaproben beruhen und bei Patienten, bei denen der Verdacht auf Vorliegen einer Demenzerkrankung, insbesondere von AD, besteht, für eine Positivdiagnose und/oder für eine negative Ausschlussdiagnose geeignet sind.

Die vorliegende Erfindung stellt eine solche Untersuchungsmethode in Form eines *in vitro* Verfahrens zur Erkennung, zur Bestimmung des Schweregrads und zur Verlaufsbeurteilung und Prognose von neurodegenerativen Erkrankungen bereit, bei dem man in einer biologischen Flüssigkeit eines Patienten, der an einer neurodegenerativen Erkrankung leidet oder bei dem Verdacht auf eine solche Erkrankung besteht, die Anwesenheit und/oder Konzentration eines physiologisch inaktiven Proadrenomedullin-Teilpeptides bestimmt und auf der Basis der festgestellten Anwesenheit und/oder Konzentration oder der Abwesenheit des bestimmten Teilpeptids Schlüsse hinsichtlich des Vorliegens, des Verlaufs, des Schweregrads oder des Erfolgs einer Therapie der neurodegenerativen Erkrankung zieht.

Vorteilhafte bzw. bevorzugte Ausgestaltungen eines Verfahrens gemäß Anspruch 1 sind in den Unteransprüchen 2 bis 10 wiedergegeben.

Der vorliegenden Erfindung liegen Überlegungen der Erfinder zugrunde, zur Verbesserung der Diagnostik von Demenz-Erkrankungen bei der Erkenntnis anzusetzen, dass die bekannten, eingangs näher erläuterten Formen der präsenilen Demenz auch - in unterschiedlichem Ausmaß - von entzündlichen (inflammatorischen) Prozessen und Endothelschäden begleitet sind, die als wesentlich für die Entwicklung, die Symptome und den Verlauf der Demenzerkrankungen angesehen werden, weshalb neurodegenerative Erkrankungen auch als neuroinflammatorische Erkrankungen angesehen werden können.

So ist Morbus Alzheimer u.a. durch das Auftreten chronischer lokaler Entzündungsreaktionen im Gehirn unter Beteiligung von verschiedenen inflammatorischen Proteinen wie Komplement-Faktoren, Akutphasen-Proteinen und proinflammatorischen Cytokinen gekennzeichnet (7, 26).

Inflammatorische Prozesse spielen auch eine Rolle bei der Entstehung von vaskulären Demenzen (VAD). Die Level von TNFα, TGFβ, IL-6 und GM-CSF (Granulozyten-Makrophagen-stimulierender Faktor) sind bei Patienten mit VAD deutlich erhöht (23, 24).

Auch bei DLB scheinen inflammatorische Prozesse eine Rolle zu spielen. So ist die Zahl der aktivierten Mikroglia-Zellen im Gehirn von Patienten mit DLB erhöht, und proinflammatorische Cytokine wie TNFα werden in bestimmten Hirnregionen wie der Amygdala und dem Hippocampus überexprimiert.

Für das Auftreten inflammatorischer Reaktionen im Gehirn von FTD-Patienten gibt es dagegen nur vereinzelte Hinweise.

Ausgehend (i) von der Hypothese, dass die mit Demenzerkrankungen verbundenen neuroinflammatorischen Prozesse zu Durchblutungsstörungen, insbesondere auch zu Mikrozirkulationsstörungen des Gehirns führen, und (ii) dass insoweit eine Ähnlichkeit mit cardiovasculären Erkrankungen besteht, die mit Durchblutungsstörungen bzw. Störungen der Mikrozirkulation (4) des Herzgewebes verbunden sind, sowie (iii) von analytischen Befunden, die zeigen, dass bei solchen cardiovasculären Erkrankungen eine erhöhten Bildung u.a. des starken Vasodilators Adrenomedullin feststellbar ist, sowie schließlich (iv) unter Nutzung der Möglichkeit, die Bildung bzw. physiologische Freisetzung von Arenomedullin zuverlässig und in klinisch valider Form mit Hilfe eines neuartigen Immunoassays der Anmelderin zu bestimmen, der die Konzentration eines midregionalen, physiologisch inaktiven Präproadrenomedullin-Teilpeptids (MR-proADM; SEQ ID NO:2) misst, untersuchten die Erfinder die Frage, ob sich auch bei Patienten mit Demenzerkrankungen, insbesondere Patienten, bei denen mit hoher Wahrscheinlichkeit Alzheimer Demenz (AD) diagnostiziert worden war, und die ansonsten an keiner bekannten, mit einer erhöhten Adrenomedullinproduktion verbundenen Erkrankung litten, im Plasma erhöhte Konzentrationen des o.g. midregionalen Proadrenomedullin-Teilpeptids (MR-proADM) feststellen lassen.

Die nachfolgend im experimentellen Teil beschriebenen Messergebnisse in EDTA-Plasmaproben von augenscheinlich gesunden Normalpersonen und Alzheimer-Patienten ergaben eine klare, diagnostisch signifikante Korrelation zwischen den für das midregionale Pröadrenomedullin-Teilpeptid (MR-proADM; SEQ ID NO:2) gefundenen Konzentrationen und dem Vorliegen von Demenzsymptomen, die zur Diagnose "wahrscheinlich Alzheimer" geführt hatten.

Obwohl die Untersuchungen bisher auf Plasmaproben von Alzheimer Patienten beschränkt waren, gehen die Erfinder davon aus, dass - möglicherweise mit unterschiedlichen typischen Konzentrationsbereichen - auch bei anderen neuroinflammatorischen Demenzformen, insbesondere bei vaskulärer Demenz (VAD) und Demenz mit Lewy-Körperchen (DLB), charakteristische Erhöhungen der MR-proADM Konzentrationen in Patientenplasmen feststellbar sein müssten. Diese Annahme wird insbesondere auch durch neuere Messungen der Anmelderin zu den Konzentrationen des Biomarkers Procalcitonin in cerebrospinaler Flüssigkeit (CSF) gestützt, die eine immundiagnostisch erkennbare Verwandtschaft der genannten Erkrankungen belegt (vgl. Offenlegungschrift DE 10 2005 034 174.8, Anmeldung vom 21.08.2005).

Das für die im experimentellen Teil beschriebenen Messungen eingesetzte Bestimmungsverfahren für MR-proADM-Konzentrationen in Patientenplasmen beruht auf einem nichtkompetitiven immunoluminometrischen Sandwichassay, der in näheren Einzelheiten in der WO 2004/090546 A1 (bzw. EP 1 488 209 A1) der Anmelderin, insbesondere in den Absätzen 5. und 6. des experimentellen Teils, sowie in (21) beschrieben wird. Auf die allgemeinen Ausführungen zur Problematik der Adrenomedullin-Bestimmung in Patientenproben und die Erläuterungen zur Assaydurchführung in der WO 2004/090546 A1 (nicht vorveröffentlicht) und in (21) wird zur Ergänzung der Ausführungen in der vorliegenden Anmeldung ausdrücklich Bezug genommen.

Adrenomedullin (ADM) ist ein aus 52 Aminosäuren bestehendes Peptidhormon, das bei seiner Biosynthese aus einem längeren Vorläuferpeptid aus 185 Aminosäuren (SEQ ID NO:1) gebildet wird. Die Biosynthese des Adrenomedullin erfolgt dabei, wie bei anderen Peptidhormonen, zuerst als Präprohormon an membran(Golgi)-gebundenen Ribosomen. Nach Abspaltung der aus 21 Aminosäuren bestehenden hydrophoben N-terminalen Signalsequenz durch Signalpeptidasen und Faltung der verbliebenen Propeptide im Lumen des endoplasmatischen Reticulums werden die Propeptide im Golgi-Apparat in Vesikel verpackt und zur Zellmembran transportiert (20). Während des Transportes erfolgt die Prozessierung der Propeptide zu reifen Hormonen durch Prohormon-Convertasen an zumeist dibasischen Aminosäuresequenzen (1). Über verschiedene Stimuli erfolgt dann die Freisetzung der physiologisch aktiven, reifen Peptide (Peptidhormone) in den extrazellulären Raum bzw. ins Plasma. Die reifen aktiven Peptide werden nach Freisetzung üblicherweise schnell durch Proteolyse inaktiviert und/oder durch Bindung an ihre Rezeptoren aus dem Kreislauf entfernt. Sie haben daher meist nur kurze physiologische Halbwertszeiten. So wurde für ADM eine Halbwertszeit von nur 22 Minuten im Plasma ermittelt (15).

Adrenomedullin wird in hohen Konzentrationen von Gefäßendothelzellen (22) im Nebennierenmark, im Pankreas, im Herzvorhof, in den Lungen, im Dünndarm (10) sowie im Gehirn (18) exprimiert. ADM weist vielfältige biologische Aktivitäten auf (eine neuere Übersicht findet sich in 2). So führt ADM zur Absenkung des Blutdrucks, zu einer verstärkten Natriumexkretion und einem Anstieg des renalen Blutflusses. ADM inhibiert außerdem die Sezernierung von ACTH (Adrenocorticotropes Hormon) der Hypophyse sowie die HCl-Sezernierung der gastrointestinalen Mucosa. Des weiteren besitzt ADM antimikrobielle Eigenschaften gegen Gram-positive und -negative Bakterien.

Der Präproadrenomedullin-Vorläufer des ADM (SEQ ID NO:1) enthält neben ADM selbst weitere Abschnitte bzw. Teilpeptide, zu denen ein weiteres aktives Peptid, das Proadrenomedullin N-terminale 20-Peptid (PAMP), gehört, das ebenfalls hypotensive, d.h. blutdrucksenkende Eigenschaften aufweist (12). Ein weniger genau untersuchtes, als Adrenotensin bezeichnetes C-terminales Teilpeptid aus 33 Aminosäuren (Aminosäuren 153-185 des PräproADM; vgl. z.B. Darstellung in 21) ist nach den bisherigen Befunden ebenfalls vasoaktiv (ein Vasokonstriktor).

Die analytische Bestimmbarkeit des ADM selbst wird durch seine kurze Halbwertszeit, durch seine autokrine bzw. parakrine Wirkung und außerdem die Maskierung des Hormons durch ein ADM-Bindeprotein (5) und seine extremen physikalischen Eigenschaften (Haftung an Gefäßoberflächen) beeinträchtigt. Eine für Routinezwecke geeignete verlässliche, reproduzierbare direkte Quantifizierung von ADM im Blut von Patienten ist deshalb nicht möglich.

Erst die Feststellung, dass im Blut auch ein stabiles pro-ADM-Fragment, das bereits oben erwähnte PräproADM 45-92 oder MR-proADM (SEQ ID NO:2; vgl. auch WO 2004/090546 A1 bzw. 21), vorhanden ist, das in einem stöchiometrischen Verhältnis zu den wichtigsten der aktiven pro-ADM-Peptide ADM und PAMP gebildet wird, jedoch inaktiv und stabil ist, so dass seine Quantifizierung mittels Immunoassay in valider, reproduzierbarer Form möglich war, brachte einen Durchbruch für die zuverlässige Bestimmung der Bildung bzw. Freisetzung von ADM durch Messung eines Blut- bzw. Plasmabiomarkers.

Die in der vorliegenden Anmeldung berichteten Ergebnisse einer signifikanten Erhöhung der messbaren Plasmakonzentration des MR-proADM bei Alzheimer Patienten können als Bestätigung der Arbeitshypothese interpretiert werden, dass eine erhöhte ADM Produktion, messbar als erhöhte MR-proADM-Plasmakonzentration, generell bei einer krankheitsbedingten Endothelschädigung bzw. Schädigung der Mikrozirkulation zu beobachten ist. Bei Demenzerkrankungen handelt es sich um eine Beeinträchtigung der Mikrozirkulation im.Gehirn, während bei cardiovasculären Erkrankungen das Herzgewebe betroffen ist.

Die auf der Basis eines relativ kleinen Kollektivs von 20 Patienten mit cardiovasculären Erkrankungen (Herzinsuffizienz) festgestellten signifikanten Erhöhungen der messbaren MR-proADM-Plasmakonzentrationen, die in der WO 2004/090546 gezeigt sind, konnten in der Zwischenzeit bei umfangreicheren Untersuchungen mit einem größeren Kollektiv von 232 Patienten mit chronischer Herzinsuffizienz (CHF; auch digestive heart failure) vollständig bestätigt werden. So waren die messbaren MR-proADM-Konzentrationen der Patienten, nachgewiesen mit einem Sandwichassay gemäß WO 2004/090546 mit einer funktionalen Assaysensitivität (Interassay CV <20%) von 0,12 nmol/l, klar mit der Schwere der jeweiligen Herzinsuffizienz korrelierbar. Während der Mittelwert für die Plasmakonzentrationen von MR-proADM bei gesunden Kontrollpersonen (264 Personen) zu 0,33 ± 0,07 nmol/l (Wertebereich 0,10 bis 0,64 nmol/l) ermittelt wurde, betrug der Mittelwert für die CHF-Patienten 0,8 ± 0,55 nmol/l und stieg mit der Schwere der Erkrankung an (klassifiziert nach der NVHA-Klassifikation mit Klassen I, II, III und IV; mittlere MR-proADM-Konzentrationen in nmol/l für Klasse I 0,41 ± 0,15; Klasse II 0,61 ± 0,31; Klasse III 0,77 ± 0,44; Klasse IV 1,35 ± 0,77). Ein Anstieg der MR-proADM-Werte erwies sich außerdem als aussagekräftiger Prognosemarker für die Überlebenserwartung eines Herzinsuffizienz-Patienten (ein Anstieg der MR-proADM-Werte erwies sich als Prognosemarker für schlechte Überlebenschancen mit einem Risikoverhältnis (hazard ratio) von 1,181 pro 0,1 nmol/l, p<0,0001).

Nachfolgend wird die Erfindung anhand von Messergebnissen und einer Figur noch näher erläutert.

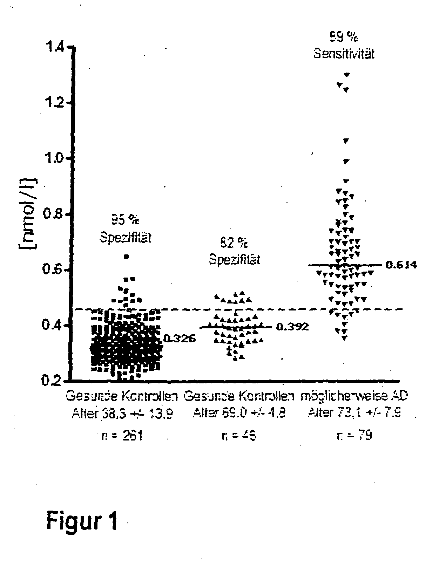

- **Figur 1**: zeigt die Ergebnisse der Messung der MR-proADM- Konzentrationen in EDTA-Plasmen von gesunden Kon- trollpersonen, wobei die Auswertung nach zwei Altersgruppen vorgenommen wurde, und von Patienten mit der Diagnose "wahrscheinlich Alzheimer Demenz".

### Experimenteller Teil

### Assaybeschreibung

Die Messung von des midregionalem pre-proADM 45-92 (MR-proADM; SEQ ID NO:2) im Plasma erfolgte mit einem immunoluminometrischen Sandwichassay im wesentlichen so wie im experimentellen Teil der WO 2004/090546 bzw. der entsprechenden EP 1 488 209 A1 bzw. in (21) oder (16) beschrieben wird.

Und zwar wurden 10 µl Probe/Kalibrator und 200 µl Tracer (markierter erster Antikörper) in die mit dem zweiten Antikörper beschichteten Röhrchen gegeben und unter Mischen (170-300 U/min) zwei Stunden bei Raumtemperatur (18-24°C) inkubiert. Dann wurde die flüssige Phase dekantiert, und die Röhrchen wurden viermal mit 1ml LUMItest Waschlösung (B.R.A.H.M.S Aktiengesellschaft, Hennigsdorf, Deutschland) gewaschen. Danach wurde die gebundene Chemilumineszenz für 1 s pro Röhrchen mit einem LB952T-Luminometer (Berthold, Wildbad, Deutschland) gemessen.

Bei Kontrollmessungen ergab sich, dass MR-proADM in EDTA-Plasmen gemessen werden sollte, da in Serum deutlich niedrigere Werte als in EDTA-Plasma erhalten werden, und auch in Heparin-Plasma und Citrat-Plasma abweichende Werte gefunden werden, die eine andere Kalibrierung erforderlich machen würden.

### Messung von MR-proADM im Plasma von gesunden Kontrollen und Patienten mit einer wahrscheinlichen Alzheimer-Demenz

Zur Ermittlung eines Bezugswerts für die Konzentration des MR-proADM wurde eine Messung in EDTA-Plasmen von 264 gesunden Kontrollpersonen durchgeführt, die weder an einer neurodegenerativen Erkrankung noch an irgendeiner anderen erkennbaren Erkrankung (cardiovasculäre Erkrankungen; schwere Infektion oder Entzündung) litten, für die bekannt ist, dass bei ihr ADM bzw. ein proADM-Teilpeptid erhöht gemessen werden kann. Für die Kontrollgruppe wurde ein Mittelwert von 0,33 ± 0,07 nmol/l (Bereich 0,10 bis 0,64 nmol/l) MR-proADM ermittelt. Bei einer Auswertung der Kontrollpersonen nach Altersgruppen wurde für die Altersgruppe 38,3 ± 13,9 Jahre ein Mittelwert von 0,337 nmol/l, und für die selektierte Altersgruppe 69,0 ± 4,8 Jahre ein Mittelwert von 0,392 nmol/l erhalten (vgl. Figur 1).

Als Patientenkollektiv dienten Patienten mit Demenzerscheinungen, bei denen die Diagnose "wahrscheinlich Alzheimer Demenz" gestellt worden war. Als Mittelwert für die Patientgruppe wurde ein Wert von 0,614 nmol/l erhalten.

Die gemessenen MR-proADM-Konzentrationen im Plasma von gesunden Kontrollen und Patienten mit einer wahrscheinlichen Alzheimer-Demenz sind in Abb. 1 gezeigt.

Die MR-proADM-Konzentration ist bei Patienten mit einer wahrscheinlichen Alzheimer-Demenz deutlich erhöht. Alzheimer-Patienten können hier mit einer Spezifität von 95% (bezogen auf jüngere gesunde Kontrollen mit einem Alter von 38,3 ± 13,9) bzw. von 82% (bezogen auf altersgematchte gesunde Kontrollen mit einem Alter von 69,0 ± 4,9) und einer Sensitivität von 89% von gesunden Kontrollen differenziert werden.

Obwohl eine erhöhte ADM-Produktion, gemessen als MR-proADM-Konzentration in Plasma, nicht nur bei Demenzerkrankungen beobachtet wird, sondern auch bei anderen Erkrankungen (Sepsis; cardiovaskuläre Erkrankungen/Herzinsuffizienz; diese sind klinisch jedoch in der Regel einfach von Demenzerkrankungen abgrenzbar) und MR-proADM daher kein gehirnspezifischer Parameter ist, eignet sich die MR-proADM-Bestimmung aufgrund der hohen Spezifität und Sensitivität bei AD-Patienten sehr gut für Zwecke der unterstützenden AD-Positivdiagnostik und insbesondere auch für die Negativdiagnostik (Ausschlussdiagnostik), wobei normale MR-proADM-Konzentrationen im Plasma eines Patienten, bei dem Verdacht auf AD besteht, eine Diagnose AD mit sehr hoher Wahrscheinlichkeit ausschließen können.

### Literatur

1. BEINFELD M. C. (1998). Prohormone and proneuropeptide processing. Recent progress and future challenges. Endocrine 8:1-5
2. BELTOWSKI J., JAMROZ A. (2004). Adrenomedullin - what do we know 10 years since its discovery? Polish Journal of Pharmacology 56: 5-27
3. BUNTON D.C., PETRIE M.C., HILLIER C., JOHNSON F., MCMURRAY J.J.V. (2004). The clinical relevance of adrenomedullin: a promosing profile? Pharmacology & Therapeutics 103:179-201
4. CHU D.Q., SMITH D.M., BRAIN S.D.(2001). Studies of the microvascular effects of adrenomedullin and related peptides. Peptides 22:1881-1886
5. ELSASSER T. H., KAHL S., MARTINEZ A., MONTUENGA L. M., PIO R., CUTTITTA F. (1999). Adrenomedullin Binding Protein in the Plasma of Multiple Species: Characterization by Radioligand Blotting. Endocrinology 140 (10): 4908-4911
6. ENDO T. (2001). A review of the biological properties and clinical implications of adrenomedulln and proadrenomedullin N-terminal 20 peptide (PAMP), hypotensive and vasodilating peptides. Peptides 22:1693-1711
7. FRANK R. A., GALASKO D., HAMPEL H., HARDY J., DE LEON M. J., MEHTA P. D., ROGERS J., SIEMERS E., TROJANOWSKI J. Q. (2003). Biological markers for therapeutic trials in Alzheimer's disease. Proceedings of the biological markers working group; NIA initiative on neuroimaging in Alzheimer's disease. Neurobiology of Aging 24: 521-536
8. GELDMACHER D. S. (2004). Dementia with Lewy bodies: diagnosis and clinical approach. Cleveland clinic Journal of Medicine 71:789-800
9. GROWDON J. H., SELKOE D. J., ROSES A., TROJANOWSKI J. Q., DAVIES P., APPEL S. et al. [Working Group Advisory Committee].(1998). Consensus report of the Working Group on Biological Markers of Alzheimer's Disease. [Ronald und Nancy Reagan Institute of the Alzheimer's Association and National Institute on Aging Working Group on Biological Biomarkers of Alzheimer's Disease]. Neurobiology of Aging 19: 109-116
10. ICHIKI Y., KITAMURA K., KANGAWA K., KAWAMOTO M., MATSUO H., ETO T. (1994). Distribution and characterization of immunoreactive adrenomedullin in human tissue and plasma. FEBS Letters 338:6-10
11. KIS B., ABRAHAM C.S., DELI M.A., KOBAYASHI H., WADA A., NIWA M., YAMASHITA H., UETA Y. (2001). Adrenomedullin in the cerbral circulation. Peptides 22:1825-1834
12. KITAMURA K., KANGAWA K., ISHIYAMA Y., WASHIMINE H., ICHIKI Y., KAWAMOTO M., MINAMINO N., MATSUO H., ETO T. (1994). Identification and hypotensive activity of proadrenomedullin N-terminal 20 peptide (PAMP). FEBS Letters 351(1): 35-37
13. KITAMURA K., KANGAWA K., ETO T. (2002). Adrenomedullin and PAMP: Discovery, Structures, and Cardiovascular Functions. Microsc. Res. Tech. 57:3-13.
14. MCKEITH I. G. (2002). Dementia with lewy bodies. British Journal of Psychiatry 180: 144-147
15. MEERAN K., O'SHEA D., UPTON P. D., SMALL C. J., GHATEI M. A., BYFIELD P. H., BLOOM S. R. (1997). Circulating adrenomedullin does not regulate systemic blood pressure but increases plasma prolactin after intravenous infusion in humans: a pharmacokinetic study. Journal of Clinical Endocrinology and Metabolism 82:95-100
16. MORGENTHALER N. G., STRUCK J., ALONSO C., BERGMANN A. (2005). Measurement of mid regional proadrenomedullin (MR-proADM) in plasma with an immunoluminometric assay. (Clinical Chemistry 51:10, 1823-1829)
17. M. GARY NICHOLLS, JOHN G. LAINBURY, LYNLEY K. LEWIS, DAVID O. MCGREGOR, A. MARK RICHARDS, RICHARD W. TROUGHTON, TIMOTHY G. YANDLE (2001). Bioactivity of adrenomedullin and proadrenomedullin N-terminal 20 peptide in man. Peptides 22 1745-1732.
18. SATOH F., TAKAHASHI K., MUR-AKAMI 0., TOTSUNE K., SONE M., OHNEDA M., ABE K., MIURA Y., HAYASHI Y., SASANO H. (1995). Adrenomedullin in human brain, adrenal glands and tumor tissues of pheochromocytoma, ganglioneuroblastoma and neuroblastoma. Journal of Clinical Endocrinolology and Metabolism 80(5):1750-2
19. SELKOE D. J. (2001). Alzheimer's disease: genes, proteins, and therapy. Physiological Reviews 81: 741-766
20. STROUD R. M., WALTER P. (1999). Signal sequence recognition and protein targeting. Current Opinion Structure Biology 9:754-9
21. STRUCK J., CHEN T., MORGENTHALER N. G., BERGMANN A. (2004). Identification of an adrenomedullin precursor fragment in plasma of sepsis patients. Peptides 25: 1369-1372
22. SUGO S., MINAMINO N., KANGAWA K., MIYAMOTO K., KITAMURA K., SAKATA J., ETO T., MATSUO H. (1994). Endothelial cells actively synthesize and secrete adrenomedullin. Biochemical and Biophysical Research Communication 201 (3) :1160-6
23. TARKOWSKI E. (2002). Cytokines in dementias. Current Drug Targets - Inflammation and Allergy 1: 193-200
24. TARKOWSKI E., LILJEROTH A. M., MINTHON L., TARKOWSKI A., WALLIN A., BLENNOW K. (2003). Cerebral pattern of pro- and anti-inflammatory cytokines in dementias. Brain Research Bulletin 61: 255-260
25. TAYLOR M.M., SAMSON W.K. (2001). Adrenomedullin and central cardiovascular regulation. Peptides 22:1803-1807
26. TEUNISSEN C. E., DE VENTE J., STEINBUSCH H. W. M., DE BRUIJN C. (2002). Biochemical markers related to Alzheimer's dementia in serum and cerebrospinal fluid. Neurobiology of Aging 23:485-508

## Patentansprüche

1. *In vitro* Verfahren zur Erkennung, zur Bestimmung des Schweregrads und zur Verlaufsbeurteilung und Prognose von neurodegenerativen Erkrankungen, **dadurch gekennzeichnet, dass** man in einer biologischen Flüssigkeit eines Patienten, der an einer neurodegenerativen Erkrankung leidet oder bei dem Verdacht auf eine solche Erkrankung besteht, die Anwesenheit und/oder Konzentration eines physiologisch inaktiven Proadrenomedullin-Teilpeptides bestimmt und auf der Basis der festgestellten Anwesenheit und/oder Konzentration oder der Abwesenheit des bestimmten Teilpeptids Schlüsse hinsichtlich des Vorliegens, des Verlaufs, des Schweregrads oder des Erfolgs einer Therapie der neurodegenerativen Erkrankung zieht.

2. Verfahren nach Anspruch 1, **dadurch gekennzeichnet, dass** das Bestimmungsverfahren ein immundiagnostisches Bestimmungsverfahren ist.

3. Verfahren nach Anspruch 1 oder 2, **dadurch gekennzeichnet, dass** man im Plasma eines Patienten ein midregionales Proadrenomedullin-Teilpeptid (MR-proADM; SEQ ID NO:2) bestimmt, das die Aminosäuren 45-92 des vollständigen Präproadrenomedullins (SEQ ID NO:1) aufweist.

4. Verfahren nach einem der Ansprüche 2 oder 3, dadruch **gekennzeichnet**, dass das immundiagnostische Bestimmungsverfahren ein Immunoassay vom Sandwichtyp ist.

5. Verfahren nach einem der Ansprüche 1 bis 4, **dadurch gekennzeichnet**, die neurodegenerative Erkrankung eine präsenile Demenzerkrankung ist, die ausgewählt ist aus einer Gruppe, die die Alzheimer Demenz (AD), Demenz mit Lewy-Körperchen (DLB), frontotemporale Demenz (FTD) und verschiedene Formen der vaskulären Demenz (VD) umfaßt.

6. Verfahren nach Anspruch 5, **dadurch gekennzeichnet, dass** es im Rahmen der Alzheimer-Diagnostik durchgeführt wird.

7. Verfahren nach einem der Ansprüche 1 bis 6, **dadurch gekennzeichnet, dass** es im Rahmen einer Multiparameter-Bestimmung durchgeführt wird, bei der gleichzeitig mindestens ein weiterer für das jeweilige Krankheitsbild aussagekräftiger biochemischer oder physiologischer Parameter bestimmt wird und bei der ein Messergebnis in Form eines Satzes von mindestens zwei Messgrößen gewonnen wird, der zur Feindiagnostik der neurodegenerativen Erkrankung ausgewertet wird.

8. Verfahren nach Anspruch 7, **dadurch gekennzeichnet, dass** im Rahmen der Multiparameter-Bestimmung neben der Bestimmung des Proadrenomedullin-Teilpeptids wenigstens ein weiterer biochemischer Parameter bestimmt wird, der aus den Gruppen der Entzündungsmediatoren, Komplementkomponenten, Cytokine, Chemokine, der Blutkoagulanzien und fibrinolytischen Faktoren, Akutphasenproteine und radikalischen Verbindungen ausgewählt ist.

9. Verfahren nach Anspruch 7 oder 8, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

10. Verfahren nach Anspruch einem der Ansprüche 7 bis 9, **dadurch gekennzeichnet, dass** die Auswertung des komplexen Messergebnisses der Multiparameter-Bestimmung mit Hilfe eines Computerprogramms erfolgt.

## Claims

1. *In vitro* method for the detection, for the determination of severity and for the monitoring and prognosis of neurodegenerative diseases, **characterized in that** the presence and/or concentration of a physiologically inactive proadrenomedullin partial peptide is determined in a biological fluid of a patient who is suffering from a neurodegenerative disease or is suspected of suffering from such a disease, and conclusions about the presence, the course, the severity or the success of a treatment of the neurodegenerative disease are drawn on the basis of the presence and/or concentration found or of the absence of the specific partial peptide.

2. Method according to Claim 1, **characterized in that** the assay method is an immunodiagnostic assay method.

3. Method according to Claim 1 or 2, **characterized in that** a midregional proadrenomedullin partial peptide (MR-proADM; SEQ ID NO: 2) which has the amino acids 45-92 of the complete preproadrenomedullin (SEQ ID NO: 1) is determined in the plasma of a patient.

4. Method according to either of Claims 2 and 3, **characterized in that** the immunodiagnostic assay method is an immunoassay of the sandwich type.

5. Method according to any of Claims 1 to 4, **characterized in that** the neurodegenerative disease is a presenile dementia which is selected from a group consisting of Alzheimer's dementia (AD), dementia with Lewy bodies (DLB), frontotemporal dementia (FTD) and various forms of vascular dementia (VAD).

6. Method according to Claim 5, **characterized in that** it is carried out as part of the diagnosis of Alzheimer's disease.

7. Method according to any of Claims 1 to 6, **characterized in that** it is carried out as part of a multi-parameter determination in which at least one further biochemical or physiological parameter informative with regard to the respective clinical picture is simultaneously determined and in which a measured result is obtained in the form of a set of at least two measured variables, which is evaluated for the fine diagnosis of the neurodegenerative disease.

8. Method according to Claim 7, **characterized in that**, as part of the multi-parameter determination, in addition to the determination of the proadrenomedullin partial peptide, at least one further biochemical parameter which is selected from the groups consisting of inflammation mediators, complement components, cytokines, chemokines, blood coagulants and fibrinolytic factors, acute-phase proteins and free radical compounds is determined.

9. Method according to Claim 7 or 8, **characterized in that** the multi-parameter determination is effected as a simultaneous determination by means of a chip technology measuring apparatus or an immunochromatographic measuring apparatus.

10. Method according to any of Claims 7 to 9, **characterized in that** the evaluation of the complex measured result of the multi-parameter determination is effected with the aid of a computer program.

## Revendications

1. Procédé in vitro pour la mise en évidence, la détermination du degré de gravité, ainsi que pour l'évaluation de l'évolution et le pronostic de maladies neurodégénératives, **caractérisé en ce que**, dans un liquide biologique d'un patient, qui est atteint d'une maladie neurodégénérative ou chez lequel est supposée une telle maladie, on détermine la présence et / ou la concentration d'un peptide partiel de la proadrénomédulline physiologiquement inactif, et, sur la base de la présence et / ou de la concentration ou de l'absence constatée/s dudit peptide partiel, on tire des conclusions en ce qui concerne la présence, l'évolution, le degré de gravité ou le succès d'une thérapie de la maladie neurodégénérative.

2. Procédé selon la revendication 1, **caractérisé en ce que** le procédé de détermination est un procédé de détermination immunodiagnostique.

3. Procédé selon revendication 1 ou 2, **caractérisé en ce que**, dans le plasma d'un patient, on met en évidence un peptide partiel de la proadrénomédulline à région moyenne (MR - proADM ; SEQ ID NO : 2), qui présente les acides aminés 45 - 92 de la proadrénomédulline complète (SEQ ID NO : 1).

4. Procédé selon l'une des revendications 2 ou 3, **caractérisé en ce que** le procédé de détermination immunodiagnostique est un dosage immunologique du type sandwich.

5. Procédé selon l'une des revendications 1 à 4, **caractérisé en ce que** la maladie neurodégénérative est une maladie démentielle, présénile, qui appartient à un groupe, qui comprend la démence de Alzheimer (AD), la démence avec corps de Lewy (DLS), la démence frontotemporale (FTD) et différentes formes de la démence vasculaire (VD).

6. Procédé selon la revendication 5, **caractérisé en ce qu'**il est exécuté dans le cadre du diagnostic de la maladie d'Alzheimer.

7. Procédé selon l'une des revendications 1 à 6, **caractérisé en ce qu'**il est exécuté dans le cadre d'une détermination à paramètres multiples, dans laquelle on détermine simultanément au moins un autre paramètre biochimique ou physiologique expressif pour l'aspect de la maladie respectivement concernée, et dans laquelle un résultat de mesure est obtenu sous la forme d'un jeu d'au moins deux grandeurs de mesure, qui est évalué pour le diagnostic précis de la maladie neurodégénérative.

8. Procédé selon la revendications 7, **caractérisé en ce que**, dans le cadre de la détermination à paramètres multiples, on détermine, en plus du peptide partiel de la proadrénomédulline, au moins un autre paramètre biochimique, qui est choisi dans les groupes des médiateurs d'inflammations, composants complémentaires, cytokine, chimiokine, des coagulants sanguins et facteurs fibrinolytiques, protéines de phase aigüe et combinaisons radicales.

9. Procédé selon revendication 7 ou 8, **caractérisé en ce que** la détermination à paramètres multiples est effectuée en tant que détermination simultanée au moyen de dispositifs de mesure selon la technologie des microplaquettes ou d'un dispositif de mesure immunochromatographique.

10. Procédé selon l'une des revendications 7 à 9, **caractérisé en ce que** l'évaluation du résultat de mesure complexe de la détermination à paramètres multiples est exécutée à l'aide d'un programme d'ordinateur.


### SEQUENCE LISTING

<110> B.R.A.H.M.S Aktiengesellschaft
<120> In vitro Verfahren zur Diagnose von neurodegenerativen Erkrankungen
<130> 4657 PCT
<150> DE 10 2005 036 094.7
   <151> 2005-08-01
<160> 2
<170> PatentIn version 3.2
<210> 1
   <211> 185
   <212> PRT
   <213> Homo sapiens
<400> 1
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Pro Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 2
   <211> 48
   <212> PRT
   <213> Homo sapiens
<400> 2